# EUROPEAN PATENT APPLICATION

(11) **EP 3 398 429 A1**
(43) Date of publication of application: **07.11.2018**
(21) Application number: 16881697.3
(22) Date of filing: 22.12.2016
(51) Int. Cl.: A01H 4/00, A01G 1/00, A01G 9/00, A01G 31/00, A01G 31/06, C12M 1/00, C12M 3/00

(54) **METHOD FOR MASS-PRODUCING PLANTS, MASS-PRODUCTION FACILITY, AND CULTURE BAG USED IN SAID METHOD AND FACILITY**

(30) Priority: 28.12.2015 JP 2015257384
(71) Applicant: KIRIN COMPANY, LIMITED, Tokyo 164-001 (JP)
(72) Inventor: ONISHI, Noboru, Tokyo 164-0001 (JP); MAMIYA, Kanji, Tokyo 164-0001 (JP); OKAWA, Hiroshi, Tokyo 164-0001 (JP)
(74) Representative: Hoffmann Eitle
(86) International application number: PCT/JP2016/088399
(87) International publication number: WO 2017/115722

(57) **Abstract**

A method for mass-producing plants includes a step of accommodating a liquid cultivation medium (12) and a plant material (13) into each of a plurality of culture bags (1) that are configured so as to be capable of standing up without assistance by being opened up from a folded state, sealing openings of the culture bags (1), and arranging the sealed culture bags (1) in a row in a predetermined cultivation space (10), and a step of cultivating the plant material within each of the plurality of culture bags (1) with an interior of each of the plurality of culture bags (1) arranged in a row in the cultivation space (10) being maintained at an environment appropriate for cultivation of the plant material.

## Description

### TECHNICAL FIELD

The present invention relates to a method for mass-production of plants by employing such as a method in which plant materials are propagated within containers.

### BACKGROUND ART

There are several per se known methods for mass-production of plants, in which a cultivation medium and a plant material are accommodated within a container, and plants are produced within the container. For example, as such production methods, a micro tuber method is per se known (for example, refer to Patent Documents 1 and 2) in which stems and leaves of a plant are propagated in a cultivation container, and subsequently tubers are induced from these stems and leaves so as to produce seed potatoes or the like; and also an organ cultivation method is per se known (for example, refer to Patent Document 3) in which stems and leaves of a plant are propagated in a cultivation container, these stems and leaves that have thus been obtained are cut randomly so as to prepare plant pieces, and then these plant pieces are budded and rooted. Furthermore there are also a so-called PPR method in which a plant material is crushed and adventitious buds are induced from the plant pieces that have thus been obtained, and an adventitious embryo method in which adventitious embryos are propagated and these are budded.

When plants are produced in large quantities by a method such as one of those described above, it is necessary to prepare a large number of containers for accommodating the plant materials and the cultivation medium. In the prior art, as one such container, a flat container made of glass or the like and having a comparatively large opening has been used (for example, refer to Patent Document 4). And, as a bioreactor for culturing plant cells and for producing secondary metabolites or for manufacturing recombinant protein drugs or the like, a cultivation tank that is mounted within a frame-like support structure and is built as a soft bag made from a polymer material and having a volume of at least around 400 liters has also been proposed (for example, refer to Patent Document 5).

### CITATION LIST

### PATENT LITERATURE

Patent Document 1: JP 2904924 B2
Patent Document 2: JP 2007-259749 A
Patent Document 3: JP 4191236 B2
Patent Document 4: WO 2012/146872 A1
Patent Document 5: JP 2014-14365 A

### SUMMARY OF INVENTION

### TECHNICAL PROBLEM

With a prior art production method that employs a large number of rigid containers, there may be a problem that a burden of setting up and operating the facility is great. For example, a great deal of space is required for housing a large number of rigid containers. Moreover, it is necessary to sterilize the containers with an autoclave device or the like before production, and this means that a large scale sterilization apparatus is needed in order to sterilize the large number of rigid containers all together. When such a sterilization apparatus cannot be employed, then there is no choice but to sterilize the large number of rigid containers sequentially in succession, but in that case it is necessary to provide secure spaces both for housing the containers before sterilization and also for housing the containers after sterilization, so that the burden imposed by the facility is great. If soft bags are used as large sized cultivation tanks, then it is necessary to install a large number of support structures in advance, so that a similar problem arises with regard to space. Moreover, if large sized cultivation tanks greater than 400 liters are employed, then there is a possibility that the losses will become rather great if these cultivation tanks become contaminated with bacteria or viruses or the like. Furthermore, if stems and leaves that require illumination for cultivation are being propagated, then, with a large sized cultivation tank on a scale such as described above, there.is a possibility that the intensity of illumination of the center portion of the tank may be insufficient.

Accordingly, the object of the present invention is to provide a method and so on, capable of reducing the burden imposed by a facility for mass production of plants.

### SOLUTION TO PROBLEM

A method for mass-producing plants according to one aspect of the present invention includes: a step of accommodating a liquid cultivation medium and a plant material into each of a plurality of culture bags that are configured so as to be capable of standing up without assistance by being opened up from a folded state, sealing openings of the culture bags, and arranging the sealed culture bags in a row in a predetermined cultivation space; and a step of cultivating the plant material within each of the plurality of culture bags with an interior of each of the plurality of culture bags arranged in a row in the cultivation space being maintained at an environment appropriate for cultivation of the plant material.

With the production method according to the aspect of the present invention described above, by opening up each culture bag so as to have capability of standing up without assistance, it is possible to allow each culture bag to stand up without the use of any support means and to thereby arrange them in a row in the cultivation space with good efficiency. Since it is possible to store the culture bags in the folded up state when they are not being used, accordingly it is possible to reduce the amount of space required for such storage. Moreover since, during sterilization of the culture bags, it is possible to manage by only opening up the openings of the culture bags somewhat so that the atmosphere for sterilization can contact the interior of the bags, accordingly, by stacking up a large number of the culture bags, it is possible to sterilize such a large number of culture bags all together in a limited space. Due to this, it is possible to reduce the burden imposed by a facility that is required for mass-production of plants.

In the production method according to the aspect of the present invention described above, in the step of cultivating, a predetermined gas may be supplied into each of the plurality of culture bags from a lower portion thereof, and the gas may be exhausted from each of the plurality of culture bags from an upper portion thereof. According to this feature, the gas such as air or carbon dioxide within the bags is repeatedly replaced with new gas, so that it is possible to maintain an environment within the bags that is appropriate for cultivation of the plant material.

In the production method according to the aspect of the present invention described above, the step of cultivating may include a step of propagating stems and leaves of the plant material. According to this feature, the production method according to the aspect of the present invention described above can be applied to production of plants by the micro tuber method or by the organ cultivation method, and it is possible to produce a large quantity of plants in an efficient manner while keeping down the burden imposed by the facility.

Furthermore, the step of cultivating may further include: a step of opening the culture bags, changing the liquid cultivation medium therein, and resealing the openings of the culture bags after the step of propagating the stems and leaves; and a step of inducing tubers from the stems and leaves after the step of resealing step. According to this feature, it is possible to induce tubers by exchanging the liquid cultivation medium while repeatedly utilizing the bags that are applied to the propagation of stems and leaves, accordingly it is possible to implement mass production of plants by the micro tuber method with good efficiency.

In the production method according to the aspect of the present invention described above, the step of cultivating may include a step of, using plant pieces cut at random as the plant material, inducing budding of the plant pieces, or causing elongation of the plant pieces. According to this feature, it is possible to produce a large quantity of plants while keeping down the cost of the facility by applying the production method of the aspect of the present invention described above to production of plants according to the organ cultivation method or according to the PPR method.

In the production method according to the aspect of the present invention described above, in the step of arranging, each level of a storage shelf that is subdivided into a plurality of levels in a vertical direction may be installed in the cultivation space, and the plurality of culture bags may be arranged in a row on each level of the storage shelf. According to this feature, it is possible to produce with good efficiency a larger amount of plants at one time in a cultivation space that is limited, by installing a larger number of bags.

In the production method according to the aspect of the present invention described above, there may be further included, a step of sterilizing each of the plurality of culture bags before the step of arranging, wherein, in the step of sterilizing, a gap defining member is inserted through the opening of each of the culture bags in the folded state, and subsequently the culture bags are arranged in a sterilizing environment so as to be stacked up. According to this feature, as compared to the case in which rigid containers are arranged in a row in a sterilizing environment, it is possible to dispose a much greater number of bags in a sterilizing environment that has limited space, so that it is possible to sterilize those bags with good efficiency by contacting their interiors with the sterilizing environment.

In the production method according to the aspect of the present invention described above, in the step of arranging, a total volume of the liquid cultivation medium and the plant material to be accommodated in each of the plurality of culture bags may be set to be equal to or less than 7 liters. By limiting the volume according to this criterion, it becomes possible for a single operator to handle the culture bags without any great trouble. Due to this, it is possible to reduce the burden of working.

In a mass-production facility according to one aspect of the present invention for cultivating a plant material that is accommodated together with a liquid cultivation medium into each of a plurality of containers that are arranged in a row in a predetermined cultivation space, a plurality of culture bags may be employed as the plurality of containers, each of the culture bags may be configured so as to be capable of standing up without assistance by being opened up from a folded state; a storage shelf subdivided into a plurality of levels in a vertical direction and a gas supply system that supplies a predetermined gas to each of the plurality of culture bags may be provided in the cultivation space; the plurality of containers may be arranged in a row on each level of the storage shelf, and are connected to the gas supply system; and each of the plurality of containers may be provided with an exhaust port that exhausts the gas from each culture bag.

With the mass-production facility according to the aspect of the present invention described above, by arranging the large number of culture bags in a row on each level of the storage shelf, and by supplying the gas from the gas supply system to each of the culture bags while exhausting the gas from each of the culture bags, it is possible to produce a large quantity of plant materials in a single production episode, while keeping the burden of the facility comparatively low.

A culture bag for plants according to one aspect of the present invention for accommodating a liquid cultivation medium and a plant material and for cultivating the plant material in an interior thereof, is configured so as to be capable of standing up without assistance by being opened up from a folded state, and provided on a side surface thereof with a port for suppling a gas and a port for exhausting the gas, and further provided, between an upper edge opening and one of the ports, with a sealing portion having a length that is at least twice the minimum length required for being sealed once.

According to the culture bag according to the aspect of the present invention described above, it is possible to provide the culture bag that is suitable for the production method and for the production facility according to the aspects of the present invention described above. And, since the length of the sealing portion is set as described above, accordingly it is possible, after having opened the temporarily sealed opening by cutting or the like, to re-seal the same culture bag at least once and to apply it to further cultivation of more plant material. Due to this, the culture bag is made to serve as a container that can be re-used, and thus it is possible to alleviate certain burdens related to manufacture of the containers and storage thereof.

### BRIEF DESCRIPTION OF DRAWINGS

Fig. 1 is a figure showing an outline of steps of a production method for plants in accordance with an embodiment of the present invention;
Fig. 2 is a figure showing an example of a culture bag;
Fig. 3 is a figure showing an example of a situation in which plants are being produced using a number of culture bags;
Fig. 4 is a figure showing an example of a single culture bag in the state of being used;
Fig. 5 is a figure showing an example of a gas supply system that is provided to a production facility;
Fig. 6 is a figure showing an example of a production method for plants according to a micro tuber method;
Fig. 7 is a figure showing an example of preprocessing when sterilizing a culture bag; and
Fig. 8 is a figure showing an example of a production method for plants according to a PPR method.

### DESCRIPTION OF EMBODIMENTS

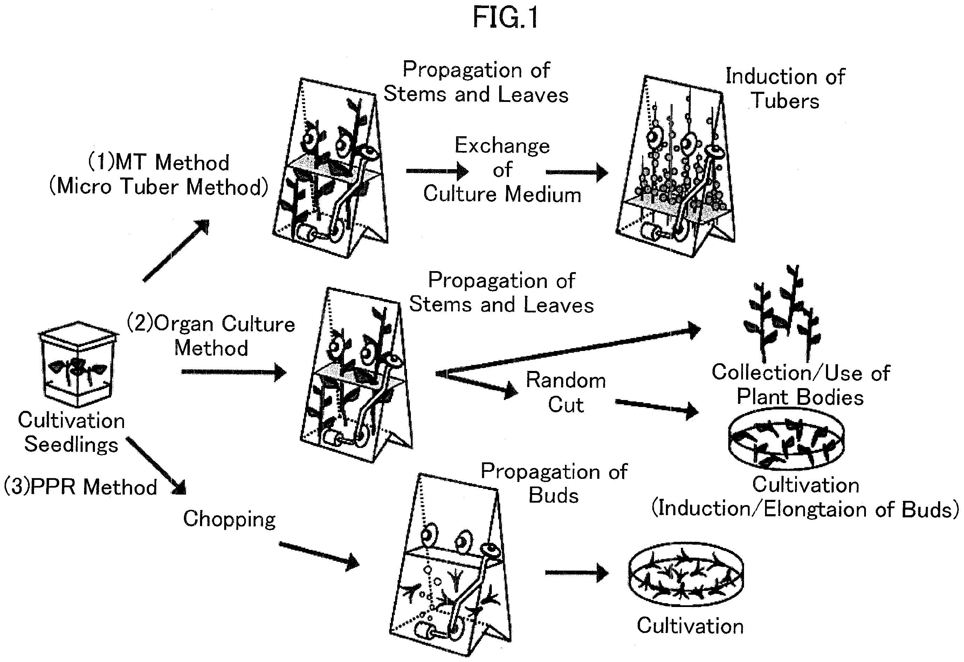

First, referring to Fig. 1, an outline of steps of a method for producing plants according to an embodiment of the present invention will be explained. It should be understood that, in this specification, "cultivating" means growing a plant material in a predetermined environment, and includes processing or operations of various types that cause the plant material to change from its initial state to a target state, such as causing stems and leaves of the plant material to propagate or to elongate, or inducing sprouting or elongation.

As examples, the production method of the present invention may be applied (1) to a micro tuber method as shown in Fig. 1 (subsequently this may sometimes be referred to as an "MT method"), (2) to an organ cultivation method, or (3) to a PPR method. Which of these methods for the plant production is adopted, may be selected as appropriate according to the plant type. For example, an MT method may be applied to the production of tubers such as potato seed tubers and so on; an organ cultivation method may be applied, for example, to the production of seedlings of dahlia, sweet potato, eucalyptus, potato, figs and so on; and a PPR method may be applied, for example, to the production of buds of types such as carnation, chrysanthemum, kalanchoe, gypsophila, petunia, gerbera, tomato, cineraria, lettuce and so on.

In each of these methods, first, plant material is produced in a predetermined cultivation container. The plant material is selected according to the objective of propagation. If the objective is the propagation of seedlings, then plant material is used that has been cultivated in a sterile environment and is free from viruses and pathogenic plant fungi. If production of some substance is the objective, then a genetically modified organism, a plant material that is infected with a recombinant virus, or the like may be used as the plant material. And, when supplying the plant material, with the MT method, a step is performed of accommodating a cultivation medium and the plant material into a predetermined cultivation container and propagating stems and leaves of the plant material therein, and subsequently a step is performed of exchanging the cultivation medium and inducing tubers from the stems and leaves. With the organ cultivation method as well, a step is performed of accommodating a cultivation medium and the plant material into a predetermined cultivation container and propagating stems and leaves of the plant material therein, and subsequently, in some cases, a step is performed of retrieving the stems and leaves that have thus been propagated as plants, while, in some cases, a step is performed of cutting up the stems and leaves at random to manufacture plant pieces, and then a step is performed of cultivating these plant pieces, inducing buds, and elongating them. Moreover, with the PPR method, after the plant material has been chopped up, a step is performed of putting the chopped up plant pieces and a cultivation medium into a cultivation container and propagating buds of the plant material, and then the buds that have thus been propagated are collected. The collected buds are then conditioned for cultivation in a greenhouse or the like by separating them into rigid cultivation containers made from resin and further cultivating them.

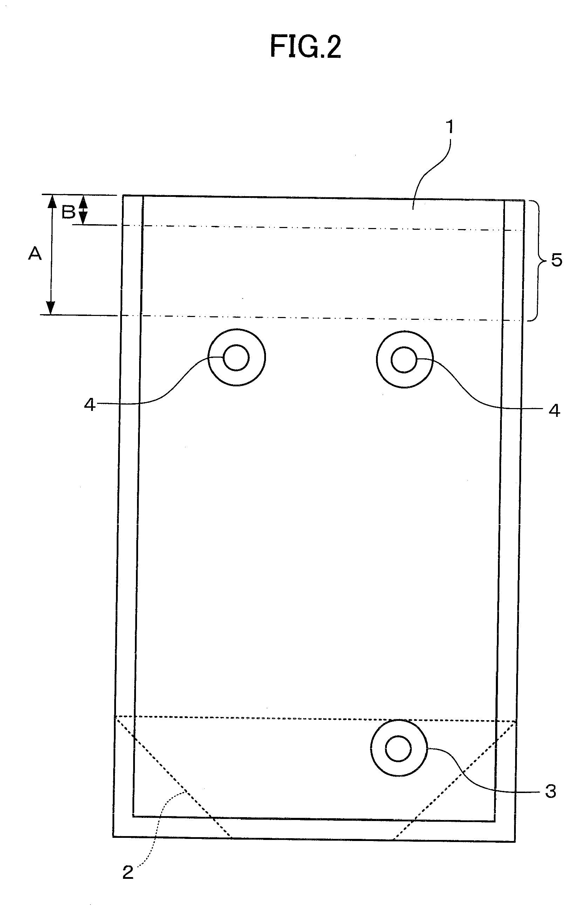

The production method of this embodiment is distinguished by the fact that, in the various steps described above, and in particular in the step of mass propagation of stems and leaves or buds or in the step of inducing tubers by the MT method, as a cultivation container, a culture bag is employed that is made from a soft resin material and that can stand up by itself without assistance. Fig. 2 shows an example of such a culture bag. This culture bag 1 is made from a compound film (of thickness 100 µm, for example) made for example from PET (polyethylene terephthalate) and CPP (non-axially stretched polypropylene), which is one example of a soft resin material, and is manufactured by forming this film into a bag shape that is higher than it is wide, and that is of a bottom gusset type with a gusset 2 being provided at its bottom portion. The resin selected for use as the raw material for the culture bag 1 is selected to be transparent to light of the wavelength region required for cultivation of the plants. The upper edge of the culture bag 1 is open over its entire width, and, with the exception of this opening, the entire periphery of the culture bag is sealed. The bottom portion of the culture bag 1 can be expanded to a certain size by opening up the culture bag 1 from its state in which the gusset 2 is folded. When this is done, the culture bag 1 becomes able to stand up by itself without assistance, and furthermore it is possible to place several culture bags 1 side by side without employing any supporting means. Moreover, when they are not in use, it is possible to store a large number of the culture bags 1 in a comparatively small space by folding up their gussets 2.

A single lower port 3 and two upper ports 4 are attached on the side of the culture bag 1. These ports 3 and 4 are for feeding gas into the culture bag 1 and for exhausting gas from the culture bag 1. As one example, the ports 3 and 4 may be made as molded products made from polypropylene resin, and may be provided by being adhered to the culture bag 1. The ports 3 and 4 may, for example, be formed in shapes to which tubes or the like can be connected. Furthermore, the region from the upper edges of the upper port portions 4 to the upper edge of the culture bag 1 functions as a sealing portion 5 for sealing the culture bag 1, after cultivation medium and plant material have been loaded into the culture bag 1. In order to make it possible to utilize the culture bag 1 several times, the dimension A of the sealing portion 5 in the vertical direction should be set to be at least twice the minimum length B that is necessary for performing sealing once.

The material for the culture bag 1 and the materials for the ports 3 and 4 are not to be considered as being limited to the examples described above. The materials for the culture bag 1 and for the ports 3 and 4 may be varied as appropriate, provided that they are capable of withstanding autoclave processing for sterilizing the culture bag 1. Moreover the capacity of the culture bag 1 may be set as appropriate, provided that it is within the range in which a single culture bag 1 containing cultivation medium and plant material can be conveniently lifted by an operator. In the present embodiment the capacity of the culture bag 1 is set to 12 L (liters) as a maximum and more desirably is set to 8 L, and the total of the cultivation medium and the plant material to be accommodated in the interior thereof, added together, should be set with an upper limit of around 60% of the capacity of the culture bag 1, in other words with an upper limit of 7 L when the capacity of the culture bag is 12 L and with an upper limit of 5 L when the capacity of the culture bag is 8 L. The ports 3 and 4 may, for example, be formed of such a size that tubes of internal diameter around 4 mm can be connected thereto.

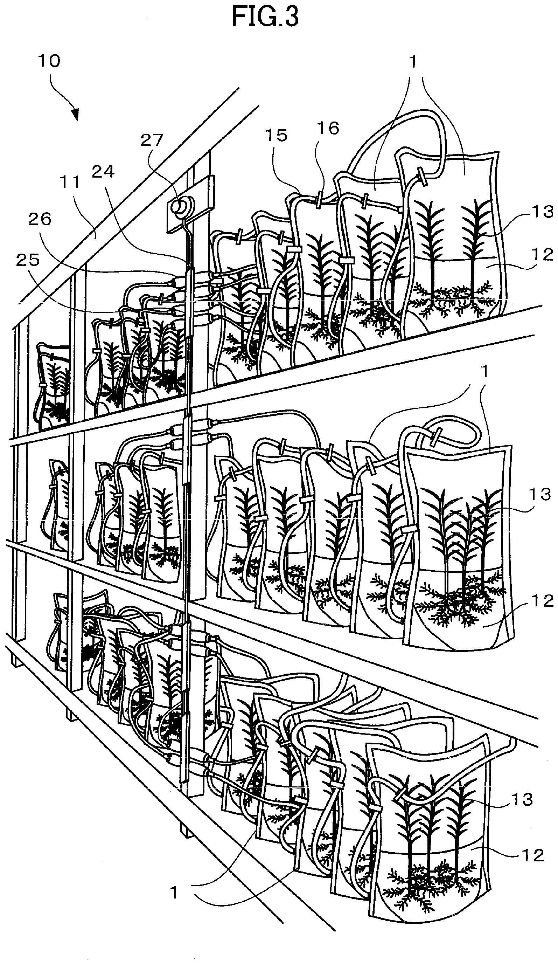

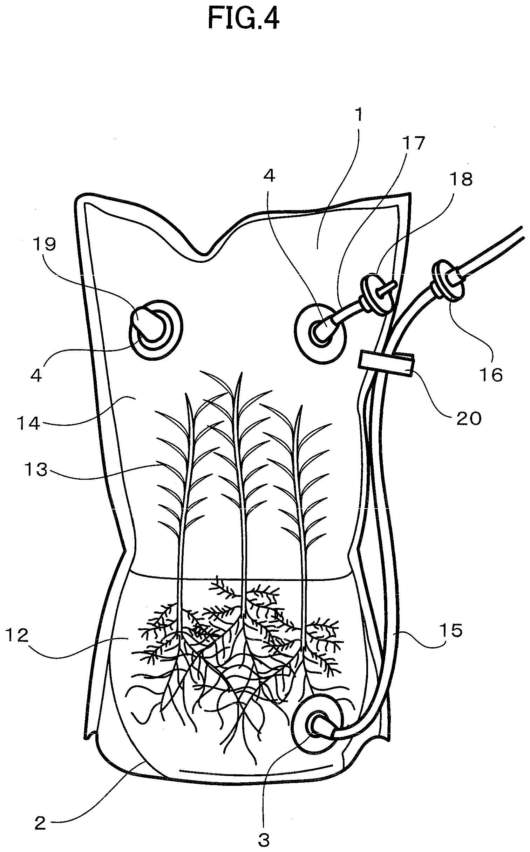

Next, with reference to Figs. 3 to 5, a summary of the process of propagation or induction of a plant material using this culture bag 1 will be explained. Fig. 3 shows an example of use of a number of the culture bags 1 in a process for producing plants, and Fig. 4 shows an example of a single culture bag in the state of being used. As shown in Fig. 3, in this embodiment, storage shelf 11 is installed in a predetermined cultivation room 10. The cultivation room 10 is divided into cultivation spaces in which physical parameters of various types that constitute a cultivation environment for plants, such as temperature, humidity, and light intensity, can be managed. The storage shelf 11 is configured so as to have a plurality of levels in the vertical direction (three levels in the shown example). The storage shelf 11 may be built to have an appropriate height in the range from the floor surface of the cultivation room 10 to the ceiling thereof, but, in consideration of the trouble during working, it is desirable to install the storage shelf 11 so that the installation surface of its uppermost level is within a height of around 1.5 m from the floor surface.

A large number of culture bags 1 are installed side by side on each level of the storage shelf 11. As shown in detail in Fig. 4, a cultivation medium 12 that has been sterilized by filtration or the like and plant materials 13 are accommodated in each of the culture bags 1, and the opening at the upper edge of each culture bag 1 is sealed in a sterile state by employing a sealing method such as heat sealing. A gaseous phase interior portion 14 is thus defined above the cultivation medium 12. A liquid cultivation medium of an appropriate composition is appropriately selected and used for the cultivation medium 12, according to the type of the plant material 13 and the production process. A ventilation tube 15 and a filter 16 are connected to the lower port 3 of the culture bag 1, a ventilation tube 17 and a filter 18 are connected to one of the upper ports 4 of the culture bag 1, and a filter 19 is connected to the other of the upper ports 4. A predetermined gas, such as for example air or carbon dioxide, is introduced into the culture bag 1 from the tubes 15 and 17, and, in exchange therewith, gas within the culture bag 1 is exhausted from the filter 19. However, it would also be acceptable for both of the upper ports 4 to be employed for exhausting gas. Moreover, it is also desirable to adjust the position in which the lower port 3 is disposed with respect to the bottom surface of the culture bag 1 and the amount of the cultivation mediao that the predetermined gas is vented from the lower port 3 into the liquid cultivation medium. By being vented within the liquid cultivation medium, the predetermined gas is efficiently supplied to the bodies of the plants, and moreover the beneficial effect may be anticipated of agitation of the liquid cultivation medium or of the bodies of the plants. The flow rate of gas to be supplied may be selected appropriately according to the type of the plants and the propagation method, but is desirably from 50 to 100 mL/min. In order to prevent the tubes 15 and 17 from becoming tangled or the like, they are fixed at appropriate positions on the culture bag 1 by using clips 20 or the like.

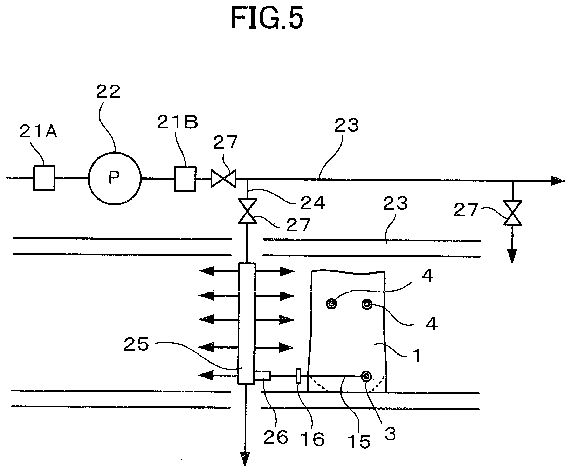

Fig. 5 shows a summary of the gas supply system for the culture bags 1. In the gas supply system shown in Fig. 5, gas (for example, air) that is sucked into a pump 22 via a pre-filter 21A is passed through a sterilization filter 21B (for example, a HEPA filter), and is supplied to a main conduit 23. If carbon dioxide is to be supplied as the gas, then a supply source such as a carbon dioxide cylinder is provided upstream of the pre-filter 21. An appropriate number of branch conduits 24 are connected to the main conduit 23, and a manifold 25 is connected to each of the branch conduits 24, corresponding to each of the levels of the storage shelf 11. A large number of tube joints 26 are connected to the manifold 25, and each of these tube joints 26 and, for example, the lower port 3 of the culture bag 1 are connected together via a tube 15 and a filter 16. In this way, gas is supplied to each of the culture bags 1. In a similar manner, if the upper port 4 is to be used for gas supply, then the tube joints 26 and the upper ports 4 are connected together via the tubes 17. On-off valves 27 are provided at appropriate positions in the main conduit 23 and in the branch conduits 24. Flow regulation valves or pressure regulation valves may also be provided instead of these on-off valves 27, or in addition thereto. According to this type of supply system, the storage shelf 11 is subdivided into a plurality of regions, and gas can be supplied only to those regions in which culture bags 1 are installed, while supply of gas can be cut off to regions in which no culture bags 1 are installed. These regions may be set as appropriate, provided that an on-off valve 27 is provided for each region. In the example of Fig. 3, the branch conduits 24 are disposed along the vertical posts of the storage shelf 11, the on-off valves 27 are connected at the base portions of these branch conduits 24, and the manifold 25 is installed to correspond to each level. However, the structure of the gas supply system is not to be considered as being limited to the layout shown in this figure; it may be varied as appropriate.

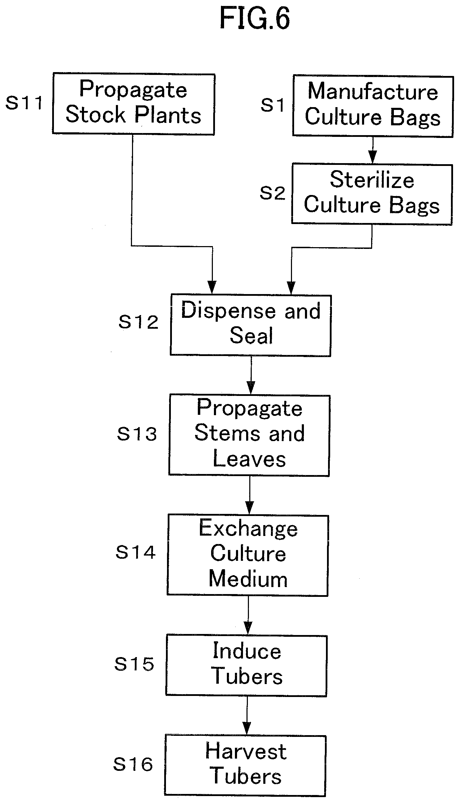

Next, examples of specific procedures in production methods for plants will be explained with reference to Figs. 6 to 8. Fig. 6 shows an example of a method for producing plants according to the MT method. In order to produce tubers of plants by the MT method, first stock plants are propagated as the first plant materials (step S11). This step is implemented by cutting virus-free seedlings at each node to obtain plant materials, and by planting the obtained plant pieces in solid cultivation media within cultivation containers and growing them. The cultivation container for this step may be smaller than the culture bag 1 shown in Fig. 2; for example, it may be a rigid container made from glass. Propagation is performed while keeping the interior of this incubator at a predetermined temperature, at a predetermined humidity, and at a predetermined illumination level.

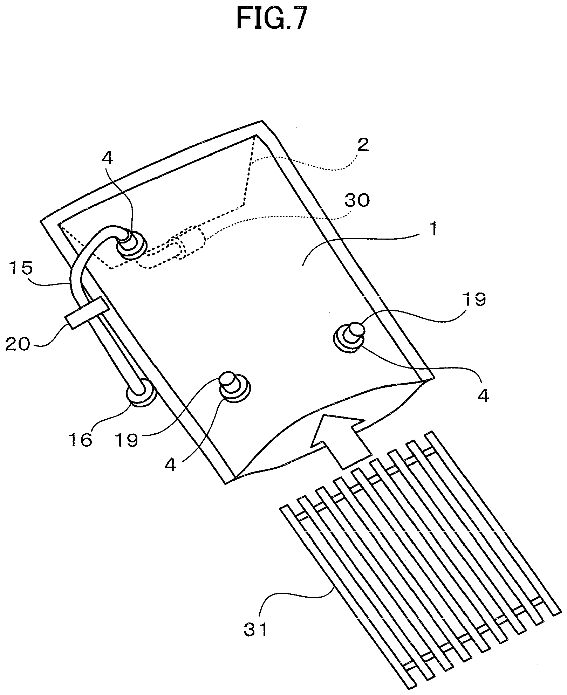

In parallel with the propagation of the stock plants, culture bags 1 are manufactured (step S1) and these are sterilized (step S2). Then preparation of the culture bags 1 is performed by attaching the tubes 15, 17 to the ports 3, 4, installing the filters 16, 18, and 19 and the clips 20, and also installing air stones 30 for aeration within the culture bags 1 (refer to Fig. 7; however, the air stones 30 may be omitted as appropriate). In short, the culture bags 1 are manufactured by attaching, to the culture bags 1, accessories of various types that need to be installed to the culture bags 1 for the process of propagating stems and leaves. Furthermore, sterilization of the culture bags 1 is performed by putting a large number of the culture bags 1 that have thus been manufactured into an autoclave device, and sterilizing those culture bags 1 all together. This sterilization is performed with the culture bags 1 in the state in which their gussets 2 are folded, but, in order to diffuse the steam properly within the culture bags 1, duckboard-like gap defining members 31 are inserted into the culture bags 1 in order to define open spaces in their interiors, as shown in Fig. 7. The large number of culture bags 1 are stacked within the interior of the autoclave device, with their openings being somewhat distended by the insertion of the gap defining members 31. Since, as compared to the volume of a rigid container made from glass or the like, the volume of the culture bag 1 in this state is much smaller, accordingly it is possible to reduce the size of the autoclave device required for the sterilization processing, and it is also possible to reduce the space required for storage of the culture bags 1, both before and after sterilization. After sterilization, the culture bags 1 are stored in a predetermined sterile environment. It should be understood that although, in Fig. 7, an example is shown in which only filters 19 are installed to the upper ports 4, it would also be acceptable to connect one or more tubes 17 to the upper ports 4, as appropriate.

After the propagation of the stock plants and sterilization of the culture bags 1 described above, next, liquid cultivation media are accommodated into each of the large number of sterilized culture bags 1, and the stock plants that have been propagated are dispensed to these portions of liquid cultivation media, and then the culture bags 1 are sealed (step S12). This operation is carried out in a sterile environment. The sealing of the culture bags 1 may be implemented, for example, by heat sealing. However, it would also be acceptable to fold the opening of each of the culture bags 1 back on itself several times, and then to squeeze this folded portion with a sealing member. Subsequently, the culture bags 1 are arranged in a row on the storage shelf 11 in the cultivation room 10, and the tubes 15 and so on are connected to a gas supply system. This completes the preparations required for propagation of stems and leaves. The culture bags 1 are relatively compact in structure and have an upper contents limit of around 5 L, and moreover the bags 1 are capable of standing up by themselves and are moderately flexible. Accordingly it is possible to arrange a large number of the culture bags 1 upon the storage shelf 11 with good efficiency. Furthermore, since the culture bags 1 are higher than they are wide, accordingly it also may be expected that the space required for installing them can be reduced.

Next, the stems and leaves are propagated within the culture bags 1 while feeding air (one example of a gas) into the culture bags 1 from the gas supply system (step S13). Due to air being supplied from the lower ports 13, the liquid cultivation medium gradually evaporates and the amount thereof decreases. During propagation, the temperature, the humidity, and the level of illumination intensity within the cultivation room 10 are kept in a state that is suitable for the propagation of stems and leaves. For example, when propagating stems and leaves of potatoes, the illumination intensity is adjusted so that the interior of the cultivation room 10 becomes bright, and the temperature is maintained at room temperature. When the stems and leaves have elongated sufficiently, next, the cultivation medium is exchanged (step S14). This step is implemented by the sealed portions at the upper edges of the culture bags 1 being opened in a sterile environment, the remaining culture medium in the interiors of the bags being discarded, sufficient liquid cultivation medium as needed for inducing micro tubers being again accommodated into the culture bags 1, and the openings of the culture bags 1 then being resealed. At this time, the stems and leaves in the culture bags 1 may be left as they are within the bags. Because the culture bags 1 are comparatively compact and also are flexible, the burden of this task of exchanging the cultivation medium is comparatively easy.

After the cultivation media have been exchanged, the culture bags 1 are again arranged upon the storage shelf 11 within the cultivation room 10, and the culture bags 1 are connected to the gas supply system again. Subsequently, tubers are induced from the stems and leaves within the culture bags 1 while feeding air into the culture bags 1 (step S15). At this time as well, the volumes of the liquid medium gradually decrease as the gas is supplied from the lower ports 3. The temperature, humidity, and light intensity conditions of the cultivation room 10 are maintained in a state suitable for the induction of tubers. For example, when potato tubers are to be induced, the illumination is turned off so that the cultivation room 10 becomes dark. When the tubers have been sufficiently induced, then the culture bags 1 are opened and the tubers accommodated therein are harvested (step S16). The tubers that have been thus obtained are packed in a state prescribed for shipment after processing such as drying and sorting, and then are shipped.

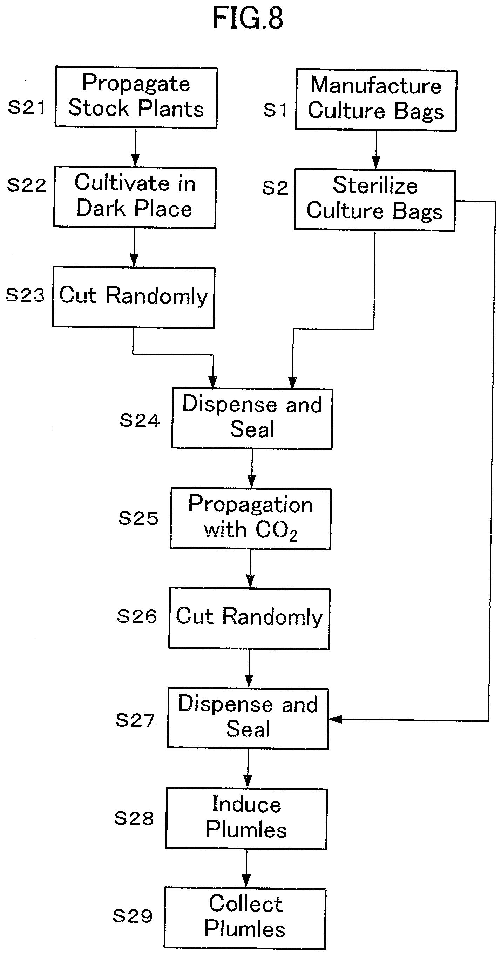

Fig. 8 shows an example of a production method for plants according to a PPR method. In order to produce plants by the PPR method, first, the stock plants are propagated as plant materials (step S21). This step is generally the same as the step S11 of Fig. 6. Next, the plant materials for propagation is planted in a solid cultivation medium within a cultivation container, and plants are further cultivated in a dark place (step S22). In this step, the plant materials are cut into lengths of two or more nodes, and are planted in the solid cultivation media, thus being caused to extend further. When the plant materials that have thus been propagated in a dark place have grown to a certain length, that plant materials are cut at random, thus manufacturing materials for propagation (step S23). In parallel with the above steps, culture bags 1 are manufactured (step S1) and these culture bags 1 are sterilized (step S2), in the same manner as in the case shown in Fig. 6. However, in this embodiment, since culture bags 1 of a plurality of types having different capacities are employed, accordingly manufacture and sterilization of the culture bags 1 is performed individually for each type of bag.

After the plant materials that have undergone propagation in a dark place have been cut at random, a liquid cultivation medium is accommodated into each of the large number of culture bags 1 that have been sterilized, the cut plant materials are dispensed into these bodies of liquid cultivation media, and the culture bags 1 are sealed (step S24). These steps are carried out in a sterile environment. The sealing of the culture bags 1 may be implemented in a similar manner to the case shown in Fig. 6. Subsequently, the culture bags 1 are arranged in a row on the storage shelf 11 of the cultivation room 10, and the tubes 15 and so on are connected to the gas supply system. This completes the preparations required for propagation of stems and leaves. The culture bags 1 that are used in this case are of smaller capacity than in the example of Fig. 6.

Next, the plant materials are propagated within the culture bags 1 while feeding carbon dioxide (one example of a gas) into the culture bags 1 from the gas supply system (step S25). In this case, the illumination intensity is adjusted so that the interior of the cultivation room 10 becomes bright, and the temperature is maintained at room temperature. When the plant materials have elongated sufficiently, next, the sealed portions at the upper edges of the culture bags 1 are opened in a sterile environment, and the elongated plants are taken out and cut up at random (step S26). Subsequently, a liquid cultivation medium for inducing budding is accommodated into each of a large number of sterilized culture bags 1, the cut plant materials are shared out into the liquid cultivation media, and the culture bags 1 are sealed (step S27). The culture bags 1 that are used in this case are of larger capacity than those used in step S24, and are of roughly the same size as those used in step S12 of the Fig. 6 example.

After the culture bags 1 have been sealed, the culture bags 1 are arranged upon the storage shelf 11 within the cultivation room 10, and the tubes 15 and so on are connected to the gas supply system. By doing this, the required preparations for inducing budding are completed. Next, buds of the plants within the culture bags 1 is induced while feeding air (one example of a gas) from the gas supply system to the culture bags 1 (step S28). In this step, the temperature, the humidity, and the level of illumination intensity within the cultivation room 10 are kept in a state that is suitable for the propagation of stems and leaves. For example, the light intensity may be adjusted so that the interior of the cultivation room 10 becomes bright, and the temperature may be maintained at room temperature. When buds have been sufficiently induced, subsequently, the culture bags 1 are opened, and the buds within them are retrieved (step S28). The buds that have thus been obtained are then shared out into rigid resin cultivation containers after processing such as drying or the like, and are cultivated further. By doing this plumules are acclimated for cultivation in greenhouses or the like, and then they are packed in a predetermined form for shipping.

It should be understood that, if plants are to be produced by employing the organ cultivation method, it is possible to propagate stock plants in a similar manner to the procedure up to step S13 of the MT method described above, to accommodate the stems thereof and cultivation media into culture bags 1, to propagate the plant stems in a bright place or in a dark place, and to retrieve and use the bodies of the plants that have thus been obtained. Alternatively, a procedure may also be adopted of propagating the plants in a dark place, cutting the bodies of the plants that have thus been obtained at random, further cultivating the cut plant pieces, inducing budding, and causing elongation. With the production of plants using this method, furthermore, instead of the stock plants of the MT method or the PPR method, it is also possible to employ differentiated totipotent cells of the plants to be produced as plant materials, and, after having propagated the obtained adventitious embryos, to accommodate the adventitious embryos and the cultivation media into the culture bags 1, and to induce buds from the adventitious embryos.

The present invention is not to be considered as being limited to the embodiments described above; various appropriate alterations and changes may be implemented, provided that, as containers, bags are employed that are configured so as to be able to stand up by themselves without assistance by being opened up from the folded state, and provided that the cultivation medium and the plant material are accommodated into those bags and the plants are cultivated in those bags. For example, it would also be possible for the culture bags 1 to be configured with gussets being provided on their side portions, in addition to gussets being provided on their bottom portions. Moreover, the shapes of the cultivation bags may be varied as appropriate, according to their intended purpose. Some of the steps of production may also be omitted as appropriate, and additional steps may be supplemented.

## Claims

1. A method for mass-producing plants, including:
a step of accommodating a liquid cultivation medium and a plant material into each of a plurality of culture bags that are configured so as to be capable of standing up without assistance by being opened up from a folded state, sealing openings of the culture bags, and arranging the sealed culture bags in a row in a predetermined cultivation space; and
a step of cultivating the plant material within each of the plurality of culture bags with an interior of each of the plurality of culture bags arranged in a row in the cultivation space being maintained at an environment appropriate for cultivation of the plant material.

2. A method for mass-producing plants according to Claim 1, wherein, in the step of cultivating, a predetermined gas is supplied into each of the plurality of culture bags from a lower portion thereof, and the gas is exhausted from each of the plurality of culture bags from an upper portion thereof.

3. A method for mass-producing plants according to Claim 1 or 2, wherein the step of cultivating includes a step of propagating stems and leaves of the plant material.

4. A method for mass-producing plants according to Claim 3, wherein the step of cultivating further includes: a step of opening the culture bags, changing the liquid cultivation medium therein, and resealing the openings of the culture bags after the step of propagating the stems and leaves; and a step of inducing tubers from the stems and leaves after the step of resealing step.

5. A method for mass-producing plants according to Claim 1 or 2, wherein the step of cultivating includes a step of, using plant pieces cut at random as the plant material, inducing budding of the plant pieces, or causing elongation of the plant pieces.

6. A method for mass-producing plants according to any one of.Claims 1 to 5, wherein, in the step of arranging, each level of a storage shelf that is subdivided into a plurality of levels in a vertical direction is installed in the cultivation space, and the plurality of culture bags are arranged in a row on each level of the storage shelf.

7. A method for mass-producing plants according to any one of Claims 1 to 6, further including a step of sterilizing each of the plurality of culture bags before the step of arranging, wherein, in the step of sterilizing, a gap defining member is inserted through the opening of each of the culture bags in the folded state, and subsequently the culture bags are arranged in a sterilizing environment so as to be stacked up.

8. A method for mass-producing plants according to any one of Claims 1 to 7, wherein, in the step of arranging, a total volume of the liquid cultivation medium and the plant material to be accommodated in each of the plurality of culture bags is set to be equal to or less than 7 liters.

9. A mass-production facility for plants, for cultivating a plant material that is accommodated together with a liquid cultivation medium into each of a plurality of containers that are arranged in a row in a predetermined cultivation space, wherein:
a plurality of culture bags are employed as the plurality of containers, each of the culture bags being configured so as to be capable of standing up without assistance by being opened up from a folded state;
a storage shelf subdivided into a plurality of levels in a vertical direction and a gas supply system that supplies a predetermined gas to each of the plurality of culture bags are provided in the cultivation space;
the plurality of containers are arranged in a row on each level of the storage shelf, and are connected to the gas supply system; and
each of the plurality of containers is provided with an exhaust port that exhausts the gas from each culture bag.

10. A culture bag for plants, for accommodating a liquid cultivation medium and a plant material and for cultivating the plant material in an interior thereof, configured so as to be capable of standing up without assistance by being opened up from a folded state, and provided on a side surface thereof with a port for suppling a gas and a port for exhausting the gas, and further provided, between an upper edge opening and one of the ports, with a sealing portion having a length that is at least twice the minimum length required for being sealed once.
